# EUROPEAN PATENT APPLICATION

(11) **EP 3 146 887 A1**
(43) Date of publication of application: **29.03.2017**
(21) Application number: 16158687.0
(22) Date of filing: 04.03.2016
(51) Int. Cl.: A61B 1/00, A61B 1/07, A61M 25/00, A61B 5/00, A61B 90/30

(54) **LIGHTGUIDE ASSEMBLY**

(30) Priority: 24.09.2015 TW 104131668
(71) Applicant: Tseng, Hsiao-Sen, 42050 Taichung City (TW)
(72) Inventor: Tseng, Hsiao-Sen, 42050 Taichung City (TW)
(74) Representative: Patentship Patentanwaltsgesellschaft mbH

(57) **Abstract**

Disclosed is a lightguide assembly comprising a main body (10); a lightguide (30) with at least a portion thereof incorporated in a side wall of the main body (10); and a light coupling seat (20) attached to an end of the lightguide (30). The lightguide (30) may be disposed within a lightguide groove (11) in a side wall of the main body (10). The coupling seat end of the lightguide (30) comprises a first portion (31) at an end portion, a narrowing section (32) connected to the first portion (31) and a second portion (33) connected to the narrowing section (32), wherein the long axis of the cross section of the lightguide (30) reduces from a first portion (31) side of the narrowing section (32) to a second portion (33) side of the narrowing section (32).

## Description

### TECHNICAL FIELD OF THE INVENTION

The present invention relates to a lightguide assembly, in particular to a duct having a lightguide, especially a catheter having a lightguide, for the transmission of a light beam into an object.

### PRIOR ART

The catheter is a tool widely used in industrial, medical applications, service industry and daily life, to introduce an object, especially a fluid into an object or a cavity, or to introduce an object or a fluid from such object or cavity. In many applications, a catheter needs to have a light-guiding function. Such applications include industrial detection, medical detection and treatments.

Recently, stimulating a human tissue or body fluids using optical energy, in order to improve the physical conditions of a person, has become a popular application in many countries. Commonly seen methods are using a probe with an optical fiber to insert into a human tissue or body fluids, followed by applying a laser beam to the insertion portion and the surroundings thereof.

In addition, various optical needles for the transmission of light beams as light sources for, such as, detections, have also been developed.

CN103901233A discloses a probe with an optical fiber. An end of the optical probe is etched to form a tip with an oval end surface. A metal coating is provided around the tip, with the tip exposed from the coating.

CN104287960A discloses an acupuncture needle with an optical fiber provided inside the acupuncture needle. An end of the optical fiber forms a tapered tip at a micro pore of the tip of the acupuncture needle, to guide a laser beam into a target position of the needle. Other micro pores are provided for the transmission of electrical and optical signals.

CN204073134 discloses a multi-channel laser treatment equipment, including eight mutually independent laser treatment channels, each including a laser light source and an optical fiber. Laser light is provided in the form of continuous or pulsed irradiations to irradiate an acupoint.

TW M493360U discloses an optical needle for intravenous irradiations. The optical needle provides a through hole at its base. An end of the through hole may be connected by a tube, to be inserted by an optical fiber, such that the optical fiber passes through the through hole. A cap is provided to seal the through hole, after the optical fiber is sterilized.

US 2014/0121538A1 discloses an assembly of an optical fiber and a metal needle, which provides a plurality of optical fiber tunnels therein. The tip of the needle forms two tilt angles so that an end of the optical fiber protruding from a second tilt angle, without protruding from the first tilt angle.

US 2014/0243806A1 discloses a hollow needle with optical fibers embedded therein. A plurality of tunnels is provided in the needle, to accommodate the plurality of optical fibers. A hub is provided to connect the plurality of optical fibers to a laser source. In the hub, a plurality of lightguides is provided, to guide laser beams from the laser source to the respective optical fibers.

WO 2014/133500A1 discloses a diagnostic probe. The probe includes a needle body provided with a plurality of tunnels to accommodate optical fibers. The respective optical fibers terminate at different longitudinal positions of the needle body, to collect diagnostic information of tissues surrounding the terminals.

Observations in the development of the conventional optical needle reveal that certain puncture needles have been designed to provide a light-guiding function. These optical needles, however, used a complicated needle body structure. Most conventional optical needles use a needle tube to support optical fiber(s) in the tube for providing the required light-guiding function. The needle tube is made of metal or other rigid materials. Forming a hollow in the needle body and assembling an optical fiber in the hollow are both difficult and time consuming. In addition, the optical fiber would occupy the full hollow portions of the needle, therefore there is no space left for the transmission of other fluid, electricity or lights in the optical needle. Moreover, the needle bodies of most optical needles are made of rigid materials, thus limiting the direction and reachable scope of the light beams so supplied.

Nevertheless, most optical needles are connected to a separate light source through an optical fiber. In order to connect the lightguide embedded in the optical needle to the optical fiber that is capable of transmitting a light beam for a certain distance, a coupler to align the lightguide and the optical fiber will be necessary. The coupler makes the optical needle system bulky and adds additional costs to the manufacture and application of the system.

### OBJECTIVES OF THE INVENTION

The objective of the present invention is to provide a novel lightguide assembly, such that the lightguide material of the lightguide assembly does not occupy the main transmission channel of the lightguide assembly.

Another objective of the present invention is to provide a lightguide assembly that may be easily coupled to a light source without the need of an optical coupler.

Another objective of the present invention is to provide a catheter, especially a flexible catheter having a lightguide.

Another objective of the invention is to provide a lightguide assembly that is easy to assemble to a light source.

Another objective of the invention is to provide a novel method for the preparation of a lightguide assembly, wherein the lightguide material of the lightguide assembly does not occupy the main transmission channel of the lightguide assembly,

### SUMMARY OF THE INVENTION

According to this invention, a lightguide assembly is provided and comprises a main body, with at least one lightguide groove in a side wall of the main body; a lightguide with at least one portion disposed within the lightguide groove; and a light coupling seat attached to an end of the lightguide. In an embodiment of the present invention, a lightguide assembly is provided and comprises a main body; a lightguide with at least a portion thereof incorporated in a side wall of the main body; and a light source coupling seat attached to an end of the lightguide. In the lightguide assemblies, the coupling seat end of the lightguide comprises a first portion at an end portion, a narrowing section connected to the first portion and a second portion connected to the narrowing section, characterized in that the long axis of the cross section of the lightguide reduces from a first portion side of the narrowing section to a second portion side of the narrowing section. The long axis may be the diameter of the lightguide. The light source coupling seat provides a junction plane for interfacing a light source and the lightguide, such that an end surface of the first portion of the lightguide is aligned with the junction plane of the light source coupling seat. The first portion and the narrowing section may be arranged in the light source coupling seat. The main body may be a tube-shaped object, especially a flexible tube. Reduction of the long axis or the diameter may be a continuous reduction or a gradient reduction.

In a particular application of the invention, the lightguide assembly is used to transmit light beams into an object, such as a human tissue, a cavity or a pouch. If the main body has a tube-shape, a first section of the tube adjacent to the light source coupling seat may comprise at least one fluid access. The first section may further comprise a coupler for coupling a plurality of fluid catheters, optical catheters and/or signal conductors. A second section of the tube away from the light source coupling seat may comprise at least one fluid access, and may have a diagonal cut to facilitate puncture purposes of the tube. The light source coupling seat may be coupled to the main body

The lightguide assembly of the present invention may connect a light source at the light source coupling seat. The light source may be an optical fiber cable or a laser head. The laser head may provide a coupling portion with a protrusion having a shape complimentary to the shape of a recess of the light source coupling seat. The coupling portion may also have a recess with a shape complimentary to the shape of a protruding portion of the light source coupling seat. The protrusion and/or recess of the coupling portion facilitates stable connection of the laser lead with the light source coupling seat and the alignment of the light emitting surface of the laser lead to the junction plane end of the lightguide. The laser head may also provide a recess for the light source coupling seat to insert in.

The present invention also discloses a preparation method of lightguide assembly. The method comprises: preparing a lightguide with a first portion on one end, a narrowing section connected to the first portion and a second portion connected to the narrowing section and extending to an other end of the lightguide. The long axis of the cross section of the narrowing section of the lightguide reduces from a first portion side of the narrowing section to a second portion side of the narrowing section. The long axis may be the diameter of the lightguide. The method further comprises: preparing a light source coupling seat with a light source junction plane; coupling the lightguide to the light source coupling seat, with the first portion and the narrowing section of the lightguide arranged in the light source coupling seat and an end surface of the first portion of the lightguide aligned with the light source junction plane, to receive light beams entering through the light source junction plane; preparing a main body with at least one lightguide groove in a side wall thereof to accommodate the second portion of the lightguide in the lightguide groove; and coupling the lightguide to the main body by arranging the second portion in the lightguide groove. Preparation of the main body and coupling of the lightguide to the main body can be completed in a single step. The junction plane may be provided in a recess, such that an end surface of the first portion of the lightguide is aligned with the recess, after the light source coupling seat is formed. The step of forming the light source coupling seat may further comprise a step of forming a recess in the light source coupling seat. The method may further include a step of sealing the lightguide groove.

The light source coupling seat may be formed by mold casting. The steps of forming the main body and connecting the lightguide to the main body may be completed by a step of injection molding. The step of sealing the lightguide groove may comprise coating a surface of the main body with a plastic material.

These and other objectives and advantages of present invention maybe clearly appreciated from the detailed description by referring to the following drawings.

### BRIEF DESCRIPTION OF THE DRAWINGS

Figure 1 shows the perspective view of the assembly of a lightguide and a light source coupling seat of the lightguide assembly of the present invention.
Figure 2 shows the cross-sectional schematic view of the assembly of the lightguide and the light source coupling seat of Figure 1.
Figure 3 is a perspective view of one embodiment of the lightguide assembly of the present invention. Figure 3A is the cross-sectional view of its main body
Figure 4 is a perspective view of another embodiment of the lightguide assembly of the present invention. Figure 4A is the cross-sectional view of its main body
Figure 5 is a perspective view of a third embodiment of the lightguide assembly of the present invention. Figure 5A is the cross-sectional view of its main body
Figure 6 is the flowchart of the method for preparation of lightguide assembly in accordance with the present invention.

### DETAILED DESCRIPTION OF THE INVENTION

The present invention provides a novel structure of lightguide assemblies and its preparation method. Although it is not intended to limit the present invention by any theory, the inventor has found that one reason that makes the conventional optical needles complicated in structure is due to the size of lightguide being too small to be installed in the optical needle easily. In addition, the size of the lightguide is not compatible with that of the optical fiber/optical fiber cable used to transmit light beams from a light source at a relatively long distance. To connect the lightguide and the optical fiber/cable with high efficiency, a coupler is needed. The inventor also found that providing a lightguide groove in the optical needle may easily solve the technical problems in the conventional art. In addition, by providing a reduction section in a lightguide or an optical fiber along the longitudinal direction, the lightguide or optical fiber so obtained would be able to support the coupling of the lightguide/optical fiber with a light source or a laser source. An invention with these and other features is thus realized.

In the followings, the lightguide assembly of the present invention will be described by referring to several embodiments thereof. It shall be appreciated that description of the embodiments serves merely to illustrate the basic structure and spirit of the present invention. The embodiments shall not be used to limit the scope of protection of this invention.

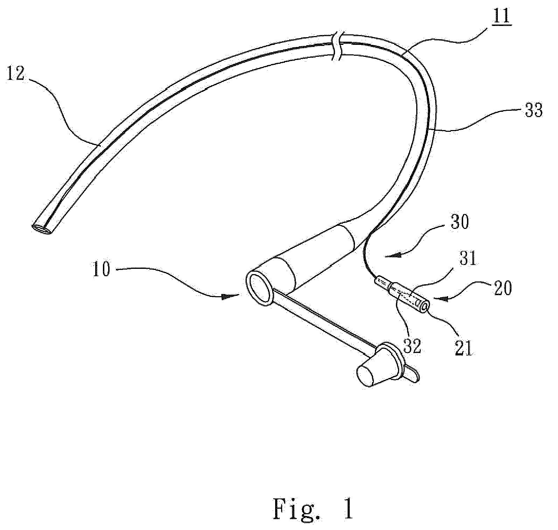

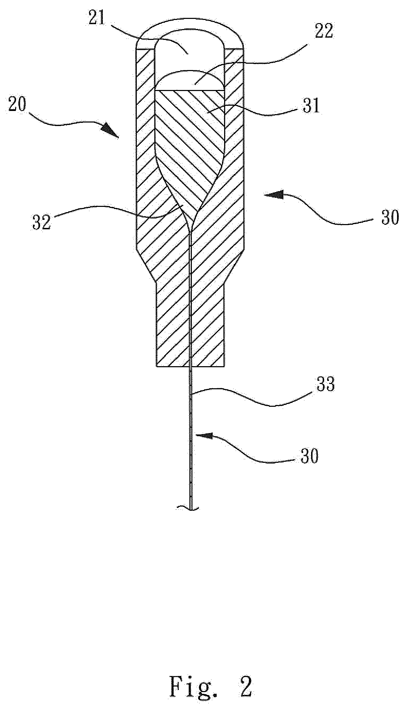

Figure 1 shows the perspective view of the assembly of a lightguide and a light source coupling seat of the lightguide assembly of the present invention and Figure 2 shows its cross-sectional view. Figure 1 also shows the associative relationship between the assembly of the lightguide and the light source coupling seat, and the main body 10. As shown, in the present invention, the light source coupling seat 20 serves to interface the lightguide 30 and a light source (not shown). The lightguide 30 may be an optical fiber, and the light source may be a laser source, for example, an optical fiber cable or a laser head. These, however, are not any technical limitation.

The lightguide 30 may be any light conductive material. Suitable materials for the lightguide 30 include: glass, plastic, metal oxides and the like. A protective film may be provided on the surface of the lightguide 30 by, for example, coating. There is no particularly limitation in the material of the protective film. The cross-sectional shape of the lightguide 30 may be elliptical, but may also be circular, square, or polygonal or in a figure-8 configuration. If necessary, the lightguide 30 can also be a beam of two or more optical fibers twisted together. In the following description, the lightguide will be described, taking the optical fiber as an example.

One of the features of the present invention is that the long axis of the cross section of the lightguide 30 reduces from its light source section (first section) 31 to its main body section (second section) 33. When an optical fiber of circular cross-section is used, the long axis is the diameter of the optical fiber. Reduction of the long axis may be a continuous reduction or a gradient reduction. Under this design, the lightguide 30 will include along its length direction: a first portion 31 at the end portion of the first section, a narrowing section 32 connected to the first portion 31, and a second portion 33 connected to the narrowing section 32, and to extend to the second section. The first portion 31 is aligned with a junction plane prepared in the light source coupling seat 20, to be coupled to the light source. The second section 33 is to be disposed within the lightguide groove 11 of the main body 10 and to extend to the distal end 12 of the main body 10. The long axis, or diameter, of the lightguide 30 starts to reduce from the junction of the first portion 31 and the narrowing section 32 and the reduction ends at the junction of the narrowing section 32 and the second portion 33. In the application of the present invention, the long axis or diameter of the first portion 31 may be between 200um to 1000um, preferably between 450um to 500um. The exact size of the first portion 31 is not any technical limitation and is preferably compatible with the size of a light emitting surface of the light source. The long axis or diameter of the second portion 33 may be between 30um to 100um, preferably between 40um to 50um. The exact size of the second portion 33 is not any technical limitation and is preferably in accordance with the actual needs. The long axis or diameter of the second portion 33 may be 40-50um, so that the lightguide 30 may be easily disposed in the side wall of the main body 10.

The second portion 33 preferably extends to the distal end 12 of the main body 10, while it may retract within the distal end or extend beyond the distal end. The light guide may be a side light optical fiber or tail light optical fiber, depending on the purpose of application.

Method for forming the narrowing section 32 of the lightguide 30 is not limited but is preferably a technique to produce a gradual or progressive reduction in the long axis of an elongated lightguide, such as an optical fiber. Suitable methods include heating stretch at high temperature, molding and other methods. Among them, stretching at high temperature produces an optical fiber with continuously reduced long axis is preferred, because the product is advantageous in transmission of light beams. Molding method forms a lightguide with gradient reduction in long axis, the advantage of which is accuracy in size of each section. Length of the narrowing section 32 is not particularly limited, but is preferably as short as possible. For example, if the long axis of the first portion 31 is 500um in length and the long axis of the second portion 33 is 50um in length, length of the narrowing section 32 can be 1mm to 5mm, so that the reduction ratio is 1/10 to 1/2 per mm. This ratio can reduce the length of the narrowing section 32, while efficient transmission of optical power is obtained. Other reduction ratios can also be used in the present invention, to obtain the same or similar effects. In addition, the reduction ratio is not necessarily linear.

The light source coupling seat 20 may be made from a rigid or flexible material. Suitable materials include plastic, silicone, resin and other plastic material. It is also possible to use metal, ceramic and other materials that are easy to process, to prepare the light source coupling seat 20. In the example of Figure 1 and 2, the top surface of the light source coupling seat 20 forms a plane. This, however, is not any technical limitation. For example, the top surface of the coupling seat 20 may be convex, concave or in another shape. The top surface may form a particular pattern or design, by using any applicable technique. It is also possible to form level difference, guide grooves or chamfer at the top surface of the coupling seat 20.

In the example of Figure 1, the light source coupling seat 20 provides a recess 21, which is open to the top surface of the coupling seat 20, whereby a cavity to accommodate a light source is formed. The bottom of the recess forms a junction plane 22. Shape of the opening of the recess 21 preferably complements a contour of the corresponding portion of a light source (not shown), so that the light source can be securely received within the recess 21. For example, if the light source is an optical cable with a diameter of about 500um, the recess 21 may provide an inner diameter of about 500um, to be plugged by the optical cable. If the light source is a laser head, the laser head may provide a protrude with a diameter of about 500um. In such a case, the recess 21 also provides an inner diameter of about 500um for coupling of the laser head. The junction plane is preferably a plane. If no recess is provided in the light source coupling seat 20, the junction plane 22 may be provided at a top surface of the coupling seat 20. A conventional mating sleeve or an adaptor or interconnector may also be used to strengthen the connection of the light source coupling seat 20 and the laser head, in place of or in addition to the recess and/or extrusion.

Those having ordinary skills in the art may appreciate that the junction plane provided in the light source coupling seat 20 is not necessarily a physical plane at the surface of an object. The junction plane may be an imaginary plane. Moreover, the junction plane may also be a top surface of the first section 31 of the lightguide 30. The embodiment may provide a light source with a recess, such as a laser head socket.

The lightguide 30 is connectable with the light source coupling seat 20 in a variety of ways. In the preferred embodiments of the invention, the lightguide 30 is connected with the light source coupling seat 20 by injection molding. In such embodiment, a tool is used to affix the assembly of the lightguide 30 and the light source coupling seat 20 as described above and the lightguide 30 is disposed in a mold that provides a cavity having a contour complimentary to the coupling seat 20. Materials for the coupling seat 20 is provided to the cavity to form the coupling seat 20. After the coupling seat 20 is formed, the assembly is removed from the mold, followed by necessary annealing. An assembly of a lightguide and a light source coupling seat is formed. In the assembly so prepared, the first portion 31 of the lightguide 30 is aligned with the junction plane of the light source coupling seat 20. If the coupling seat 20 has a recess 21, the first portion 31 of the lightguide 30 is aligned with the recess 21 of the coupling seat 20, such that an end surface of the first portion 31 is aligned with the light emitting surface of the light source to be accommodated in the recess 21 of the coupling seat 20.

In the preferred embodiments of the invention, the main body 10 may be made from any material but is preferably by a flexible material. Suitable materials include metal, ceramic, plastics, glass, or carbon fiber. The lightguide groove 11 may be formed in advance in the body 10, the inner diameter of the lightguide groove 11 of the main body 10 shall be sufficient to accommodate a portion of the lightguide 30. For example, the inner diameter of the lightguide groove 11 is preferably greater than the diameter of the lightguide 30 to embed the lightguide 30 in the lightguide groove 11, but it is acceptable if the lightguide 30 protrude from the side of the main body 10.

Suitable main body 10 of the present invention include a flexible tube, such as those used in industrial, medical applications, service industry and household. In a preferred embodiment of the present invention, the distal end 12 of the main body 10 may have a diagonal cut, to facilitate puncture function of the main body 10. The shape of the cut is not limited but is preferably a shape easy to produce.

In other preferred embodiments of this invention, the lightguide groove 11 is provided in the wall of the tube, extended along the longitudinal direction of the main body 10. In these examples, the main body may be used to transport other fluid or to accommodate a medium for electricity or signal transmission. Method for forming the lightguide groove 11 is not limited. Any known art may be used to prepare the lightguide groove. Applicable methods include casting, etching, laser cutting, forging, heat extension etc., as long as an open groove extended along the longitudinal direction of the main body 10, sufficient to accommodate a part of the lightguide 30 is formed in the main body 10. However, in the preferred embodiments of the present invention, the step of forming the lightguide groove 11 is not necessary; the assembly of the lightguide and the light source coupling seat is coupled to the main body 10 in the formation of the main body

For example, if the main body 10 is made by injection molding, the assembly of the lightguide 30 and the light source coupling seat 20 is disposed in a mold that provides a cavity having a contour complimentary to the main body 10. A plurality of lightguide-light source coupling seat assemblies, or an assembly of a plurality of lightguides and a single light source coupling seat, can be disposed within the cavity of the mold. Materials for the main body 10 is then provided to the cavity to form the main body 10. After the main body 10 is formed, the assembly is removed from the mold, followed by necessary annealing. A lightguide assembly is formed. In the lightguide assembly so prepared. The lightguide assembly so produced includes the main body 10, at least one lightguide 30 disposed on the wall or the side wall of the main body 10, and at least one light source coupling seat connecting to each lightguide 10. The first portion 31 of each lightguide 30 is aligned with the junction plane of the respective light source coupling seats 20. If the coupling seat 20 has a recess 21, the first portion 31 of the lightguide 30 is aligned with the recess 21 of the coupling seat 20, such that an end surface of the first portion 31 is aligned with the light emitting surface of the light source to be accommodated in the recess 21 of the coupling seat 20.

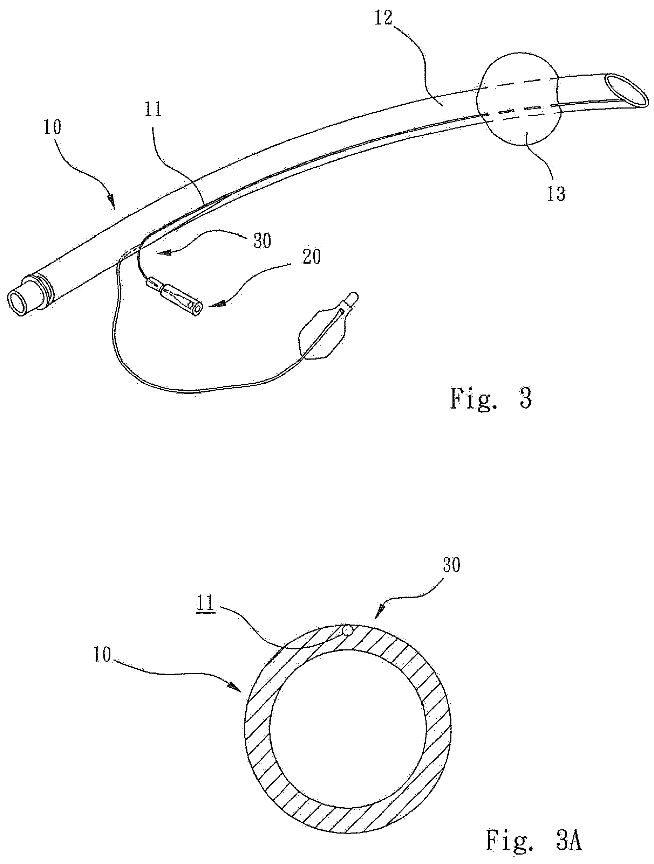

Figure 3 is a perspective view of one embodiment of the lightguide assembly of the present invention. Figure 3A is the cross-sectional view of its main body. The figures show an application example of the invented lightguide assembly in medical catheters. As shown in Figure 3, the main body 10 has a tube-shape and can be an endotrachea tube. The main body 10 can also be a nasogastric tube, etc. An assembly of the lightguide 30 and the light source coupling seat 20 is provided on the main body 10. The first section 11 of the main body 10 adjacent to the light source coupling seat 20 may include at least one fluid access. The first section 11 may further include a coupler for coupling a plurality of fluid catheters, optical conductor and/or signal conductor. In the embodiment of Figure 3, the proximal end of the tube further includes a bubble 13 for compressing air into the distal end 12 of the tube 10 emoting from the light source coupling seat 20. The distal end 12 may also include at least one fluid access. The distal end 12 of the tube may have a diagonal cut to facilitate puncture purposes of the tube. In the application example shown in Figure 3, optical powers can be introduced to the distal end 12 of the tube 10, to irradiate human tissues at the distal end 12 or the surroundings along the tube 10. Figure 3A shows the coupling of the main body 10 and a lightguide 30, but as is well known, the wall of the tube 10 may be disposed with more than one lightguide 30.

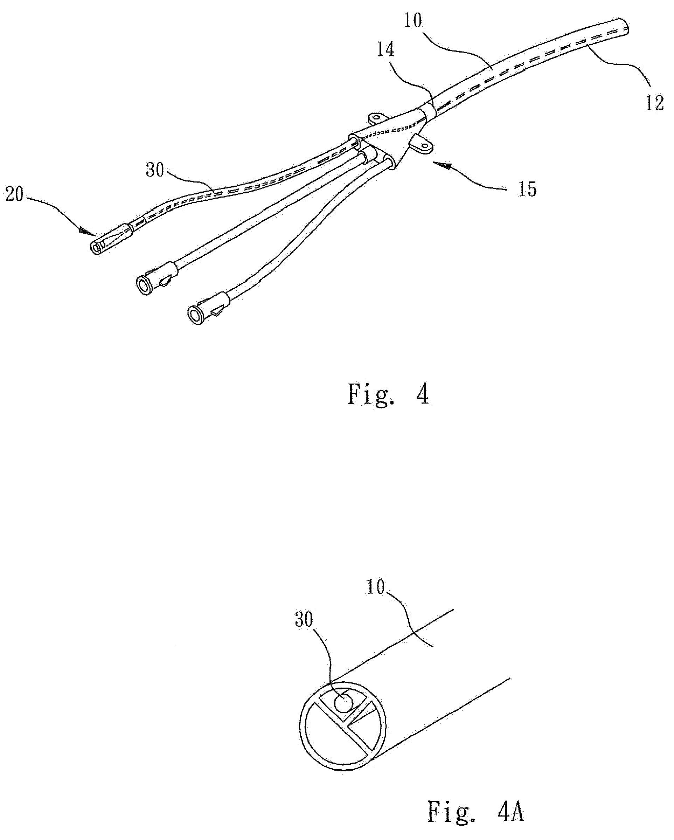

Figure 4 is a perspective view of another embodiment of the lightguide assembly of the present invention. Figure 4A is the cross-sectional view of its main body. The figures show another application example of the invented lightguide assembly as a medical catheter. As shown in Figure 4, the main body 10 is a tube and can be a central venous catheter, peripherally inserted central catheter, hemodialysis catheter, etc. An assembly of the lightguide 30 and the light source coupling seat 20 is provided on the main body 10. The proximal end 14 of the main body 10 adjacent to the light source coupling seat 20 may include at least one catheter coupler 15 for coupling a plurality of fluid catheters, optical conductors and/or signal conductors. The assembly of the lightguide 30 and the light source coupling seat 20 is inserted in one of the cavities. The distal end 12 of the tube 10 remoting from the light source coupling seat 20 may include at least one other fluid access. The second section of the tube may have a diagonal cut to facilitate puncture purposes of the tube. In the application example shown in Figure 4, optical powers can be introduced to the distal end 12 of the tube 10, to irradiate body fluids at the distal end 12 or the surroundings of the tube 10, such as human blood. Figure 4A shows the assembly of the main body 10 and the lightguide 30. The interior of the tube 10 is divided into three channels, wherein the lightguide 30 only occupies one channel.

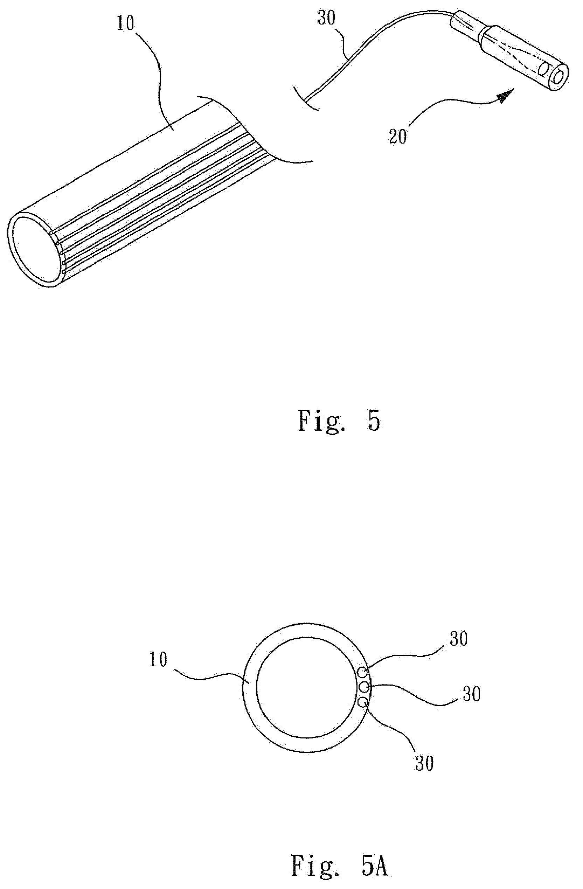

Figure 5 is a perspective view of the third embodiment of the lightguide assembly of the present invention. Figure 5A is the cross-sectional view of its main body. The figures show the application example of the invented lightguide assembly used as other medical catheters. As shown in Figure 5, the main body 10 is a tube and can be a surgical drain, chest tube, urinary catheter, etc. A plurality of lightguides 30 is provided in the main body 10, with each lightguide 30 connected to a corresponding light source coupling seat 20. Of course, it is also allowed to connect all the lightguide to a single light source coupling seat. In the application example shown in Figure 5, optical powers can be introduced to the distal end 12 of the tube 10, to irradiate human tissues or body fluids at the distal end 12 or the surroundings of the tube 10. Figure 5A shows the assembly of the main body 10 and a plurality of lightguides 30.

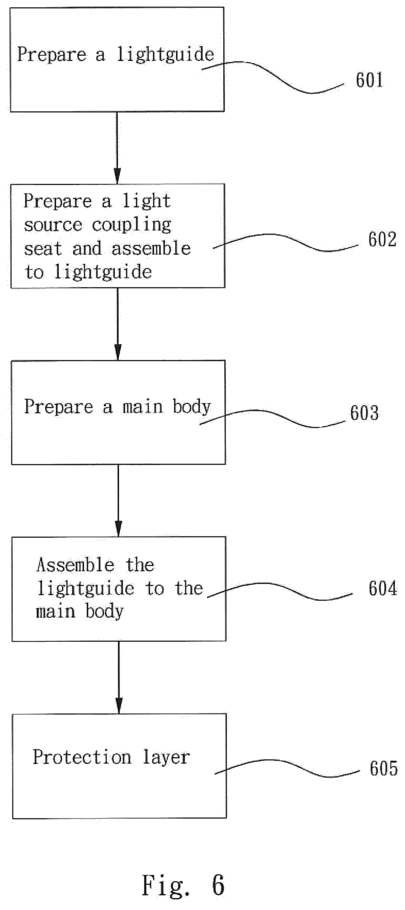

In the followings, a method for preparation of the lightguide assembly of the present invention will be described. Figure 6 shows the flowchart of the method for preparation of a lightguide assembly in accordance with one embodiment of the present invention. As shown, in the preparation of the lightguide assembly of the present invention, firstly at 601 a lightguide 30 is prepared. An end of the lightguide 30 has a first portion 31, a narrowing section 32 connected to the first portion 31, and a second portion 33 connected to narrowing section 32 and extend to the second section 33 on the other end of the lightguide. The long axis of the cross section of the lightguide 30 reduces from a first portion end of the narrowing section to a second portion end of the narrowing section. The long axis may be the diameter of the lightguide. The reduction may be a gradual reduction or a gradient reduction. Thereafter, at 602 a light source coupling seat 20 with a light source junction plan 22 is prepared. The first portion 31 and the narrowing section 32 of the lightguide 30 is positioned in the light source coupling seat 20. Assemble the lightguide 30 to the light source coupling seat 20,with an end surface of the first portion 31 of the lightguide 30 aligned with the junction plane 22, in order to receive light beams entering through the junction plane 22. At 603 a main body 10 is prepared. At least one lightguide groove 11 is provided in a side wall of the main body 10, with the second portion 33 of the lightguide 30 disposed in the lightguide groove 11. At 604 the lightguide 30 is assembled to the main body 10. In an embodiment of this invention, the preparation of the main body 10 and the connection of the lightguide 30 to the main body 10 are completed in a single step, such as a injection molding step, to form the main body 10 and the lightguide 30 can be integrally, thus omitting the step of producing the lightguide groove 11.

At step 605, a protection layer is coated on the surface of the main body 10 to cover both the main body 10 and the lightguide groove 11 and to fill any clearance between the lightguide 30 and the lightguide groove 11. Preparation of the invented lightguide assembly is thus completed. However, if the main body 10 and the lightguide 30 are integrally formed, step 605 can be omitted.

In the above embodiment of the present invention, the junction plane is provided in a recess and an end surface of the first portion of the lightguide is aligned with the recess after formation of the light source coupling seat. Thus, the step of forming a light source coupling seat may further include the formation of a recess in the light source coupling seat. However, if the light source coupling seat 20 does not provide a recess 21 but, instead, a light source junction plane at the top surface of the coupling seat 20, in such an embodiment, since a narrowing section 32 that is useful in coupling the optical fiber 30 to an optical fiber cable has been provided, simply irradiating the junction plane with a commercially available laser source, such as a laser pointer, would cause the transmission of light power to the second section 33 of the lightguide 30. If the recess 21 is necessary, the recess 21 may be formed at step 604, or in a later step by, such as, milling or lasering process. In such a step, the junction plane may also be processed to form required optical shape or characters.

In the above steps, the light source coupling seat 20 is preferably formed by molding, such as injection molding. In addition, in order to maintain the relative positions of the lightguide 30 and the main body 10, or the relative positions of the lightguide 30 and the junction plane 22, certain positioning tools may be used. The tools will become part of the main body 10 or coupling seat 20 after formation of the main body 10 and the coupling seat 20.

As described above, the present invention provides a new structure for the lightguide assembly. The lightguide assembly can be prepared easily. The lightguide assembly provides a lightguide groove and is easy to produce and assemble. The lightguide used in this invention is connectable with a variety type of light sources, without the need of an additional coupler. The present invention provides a lightguide assembly that is easy to produce and can be effectively utilized in existing catheters, cannulas, etc., to provide light illumination applications.

## Claims

1. A lightguide assembly, comprising:
a main body with at least one lightguide groove in a side wall of the main body;
a lightguide with at least a portion thereof disposed within the lightguide groove; and
a light source coupling seat attached to an end of the lightguide;
wherein the coupling seat end of the lightguide comprises a first portion at an end portion, a narrowing section connected to the first portion and a second portion connected to the narrowing section; **characterized in that** a long axis of a cross section of the lightguide reduces from a first portion side of the narrowing section to a second portion side of the narrowing section.

2. A lightguide assembly, comprising:
a main body;
a lightguide with at least a portion thereof incorporated in a side wall of the main body; and
a light source coupling seat attached to an end of the lightguide;
wherein the coupling seat end of the lightguide comprises a first portion at an end portion, a narrowing section connected to the first portion and a second portion connected to the narrowing section; **characterized in that** a long axis of a cross section of the lightguide reduces from a first portion side of the narrowing section to a second portion side of the narrowing section.

3. The lightguide assembly according to claim 1 or 2, wherein reduction of the long axis is a continuous reduction or a gradient reduction.

4. The lightguide assembly according to claim 1 or 2, wherein the long axis is diameter of the lightguide.

5. The lightguide assembly according to claim 4, wherein reduction of the diameter is a continuous reduction or a gradient reduction.

6. The lightguide assembly according to claim 1 or 2, wherein the light source coupling seat provides a junction plane for interfacing a light source; and wherein the first portion end of the lightguide is aligned with the junction plane of the light source coupling seat.

7. The lightguide assembly according to claim 1 or 2, wherein the first portion and the narrowing section of the lightguide is accommodated in the light source coupling seat.

8. The lightguide assembly according to claim 1 or 2, wherein the main body has a tube-shape.

9. The lightguide assembly according to claim 8, wherein the main body is a flexible tube.

10. The lightguide assembly according to claim 1 or 2, wherein the main body has a tube shaped, a first section of the main body adjacent to the light source coupling seat 20 further includes a coupler for coupling a plurality of fluid catheters, optically conductors and/or signal conductors.

11. A method for preparation of a lightguide assembly, comprising the steps of:
preparing at least a lightguide with a first portion on one end , a narrowing section connected to the first portion and a second portion connected to the narrowing section and extending to another end of the lightguide;
preparing a light source coupling seat with a light source junction plane;
coupling the lightguide to the light source coupling seat, with the first portion and the narrowing section of the lightguide disposed in the light source coupling seat and an end surface of the first portion end of the lightguide aligned with the junction plane, to receive light beams entering through the junction plane;
preparing a main body; and
coupling the second portion of the lightguide in a side wall of the main body;
**characterized in that** the long axis of the cross section of the lightguide reduces from a first portion side of the narrowing section to a second portion side of the narrowing section.

12. The method according to claim 11, wherein the long axis is diameter of the lightguide.

13. The method according to claim 11, wherein the light source coupling seat is formed by mold casting.

14. The method according to claim 11, wherein preparation of the main body and coupling of the lightguide to the main body are completed in a single step.

15. The method according to claim 14, wherein the step of forming the main body and coupling the lightguide to the main body is completed by an injection molding.

16. The method according to claim 11, further including a step of forming a recess in the light source coupling seat, wherein the junction plane is provided in the recess, such that the end surface of the first portion of the lightguide is aligned with the recess, after the light source coupling seat is formed.

17. The method according to claim 11, further including a step of coating a surface of the main body with a plastic material after the assembly of the main body and the lightguide.
